# EUROPEAN PATENT SPECIFICATION

(11) **EP 1 889 064 B1**
(45) Date of publication and mention of the grant of the patent: **04.11.2009**
(21) Application number: 06742449.9
(22) Date of filing: 02.06.2006
(51) Int. Cl.: G01N 33/543, B01L 3/00, B81B 3/00

(54) **SENSOR SYSTEM WITH ACTUATED STRUCTURE RELEASING A COLOURED INDICATOR**
SENSORSYSTEM MIT EINEN FARBINDIKATOR FREISETZENDER ANGETRIEBENER STRUKTUR
SYSTEME DE CAPTEUR A STRUCTURE ACTIONNEE DE LIBERATION D'UN INDICATEUR DE COULEUR

(30) Priority: 02.06.2005 DK 200500807; 07.12.2005 US 742877 P
(43) Date of publication of application: 20.02.2008
(73) Proprietor: Danmarks Tekniske Universitet, 2800 Lyngby (DK)
(72) Inventor: BOISEN, Anja, DK-3460 Birkerød (DK); HÄFLIGER, Daniel, DK-1123 Copenhagen K (DK); KELLER, Stephan, DK-2400 Copenhagen NV (DK)
(74) Representative: Plougmann & Vingtoft A/S
(86) International application number: PCT/DK2006/000313
(87) International publication number: WO 2006/128472

(56) References cited:
- WO-A-01/33226
- US-A1- 2003 092 016
- BALLER M K ET AL: "A CANTILEVER ARRAY-BASED ARTIFICIAL NOSE" ULTRAMICROSCOPY, AMSTERDAM, NL, vol. 82, no. 1-4, February 2000 (2000-02), pages 1-9, XP001011508 ISSN: 0304-3991
- WATSON J ET AL: "Gas monitoring instrument utilising fibre optic, piezoelectric and gas-sensitive polymer techniques" SENSORS AND ACTUATORS B, ELSEVIER SEQUOIA S.A., LAUSANNE, CH, vol. 34, no. 1-3, August 1996 (1996-08), pages 323-327, XP004077999 ISSN: 0925-4005

## Description

### FIELD OF THE INVENTION

The invention relates to a sensor system for detecting a target substance. The sensor system comprising a micro-mechanical component, or flexible structure, separating a first and second fluid system, moreover the invention relates to a sensor system that may be operated without use of a power source.

### BACKGROUND OF THE INVENTION

A sensor system comprising a flexible structure, which is mechanically actuated upon detection of a target substance is typically based on a cantilever or membrane type flexible structure. Cantilever-based sensors have been used to monitor different physical and chemical processes by transducing changes in temperature, mass, electromagnetic field or surface stress into a mechanical response. Cantilever-based sensors have a wide range of applications in real-time local monitoring of chemical and/or biological applications. Traditionally, cantilevers used in cantilever-based bio/chemical detection are micrometer-sized cantilevers fabricated in silicon and designed for atomic force microscopy (AFM) imaging. However, recently a new type of cantilever-based sensor has emerged, this new type being polymer-based.

The working principle of a cantilever sensor is in one form based on the optical leverage principle including a laser and a light sensitive detector, and is in another form based on a flexible structure with integrated piezoresistive strain gauge means for read-out. In the first form the sensor requires a laser to operate, and both systems require a power source to drive the laser and the stain gauge, as well as additional electronic equipment.

In WO 03/022731 a polymer-based flexible structure with integrated sensing/actuator is disclosed. By incorporating a gold resistor in an SU-8 based polymer structure an SU-8 based cantilever sensor, which is almost as sensitive to stress changes as a silicon-based cantilever with piezo-resistive read-out is achieved.

In WO 01/33226 a sensor system for detecting a target substance in a reference liquid comprising a functionalized measurement cantilever. A detector unit determines the difference in the deflection of the cantilever. WO 01/33226 also disclosed a container comprising a bendable, micromechanical lid. By exposing the lid to the target substance the lid automatically bends such that the container opens.

The presence of a laser, a power source as well as external detector equipment render devices of the prior art expensive and possibly complicated to use.

### SUMMARY OF THE INVENTION

In a wide variety of applications it is desirable to perform measurements where the result is simple to readout and where the sensor may be operated without a power source.

The present invention seeks to provide an improved sensor that preferably alleviates, mitigates or eliminates one or more disadvantages of the prior art, singly or in any combination.

Accordingly there is provided, in a first aspect, a sensor system comprising:
at least a first fluid system,
a second fluid system containing an indicator substance, the indicator substance being a coloured substance or a colour inducing substance,
a flexible structure separating the at least first fluid system and the second fluid system, the flexible structure having a first surface facing the at least first fluid system, the first surface being functionalized to interact with at least one target substance in a fluid present in the at least first fluid system, the flexible structure having a second surface facing the second fluid system,
wherein the flexible structure upon exposure to the target substance releases energy stored in the flexible structure resulting in a mechanical actuation of the flexible structure, the mechanical actuation of the flexible structure resulting in a release of the coloured indicator substance either into the first fluid system or into a third fluid system connected to the second fluid system, or resulting in a colouring of a substance released either into the first fluid system or into a third fluid system connected to the second fluid system.

The sensor system is a system facilitating non-complicated use as well as simple and direct readout of a presence of a target substance. The sensor system may be an indicator system. The target substance may either be in the form of a fluid or an analyte or be in the form of a substance added to or mixed with a fluid or an analyte, the fluid typically being a liquid or a gas. Fluid may be loaded into the sensor system, passed over one or more detection areas, in the situation a positive detection is obtained, a direct readout is provided by a release of the indicator substance from an actuation of the flexible structure.

The readout of the sensor system is obtained from detecting the release of the indicator substance either into the first fluid system or into a third fluid system connected to the second fluid system. The indicator substance is a coloured or colour inducing substance, which enables that the readout of the sensor system may be obtained by the human eye, e.g. either directly or by use of a light microscope. It is an important aspect of the invention that readout may be done with the human eye, nevertheless may the readout in certain embodiments be done by or assisted by means of a detector. The detector may be, or be part of a device attached to the sensor system, alternatively embodiment may be envisioned where the detector is an Integral part of the sensor. The readout may e.g. be performed by a light sensitive semiconductor device, such as photodiodes or a CCD. The light sensitive detector may also be connected to the sensor system via a waveguide, configured so that any light emitted as a consequence of the released indicator substance may be guided by the waveguide and detected at an output coupling of the waveguide, e.g. by means of a CCD.

Further advantages of the present invention may include that the sensor may exhibit long or even unlimited operation time, it may be produced at a low cost, that no, or only, little maintenance may be needed, and a large variety of possible analytes/target substances may be detected.

Fluid may be introduced into the at least first fluid system in a number of ways. The fluid may be loaded by human hand or may be automatically loaded, such as by means of an automated droplet injection from an inkjet-type device. Fluid may be introduced directly into the first fluid system, or the first fluid system may comprise an inlet or may be connected to a reservoir or an external fluid system. The sensor may also be dipped into a liquid for testing the presence of a given target substance in the liquid, or placed in an ambient atmosphere to test the presence of a target substance in the ambient atmosphere.

The at least first fluid system may be a microfluidic system, i.e. a system where the fluid channel(s) is/are in the millisize or microsize range, such as between 1 micron to 10 millimetre. Different parts of the system may be dimensioned based on a compromise between handling minimal fluid quantities, and providing a readout large enough for easy detection.

The at least first fluid system may be connected to a pumping system for forced flow of the fluid.

The at least first fluid system is connected to a second fluid system, the second fluid system containing the indicator substance. The volume of the second fluid system may be in the range of 1 microlitre to 1 millilitre.

A micro-mechanical component is placed in the area separating the first fluid system and the second fluid system, the micro-mechanical component being in the form of a flexible structure. The flexible structure releases stored energy upon exposure to the target substance resulting in a mechanical actuation of the flexible structure. The energy release may be in the form of stored chemical energy that is converted to mechanical energy, thereby enabling actuation without use of a power source, i.e. enabling autonomous actuation. Embodiments of mechanical actuation of the flexible structure are described in the following. The energy release and the resulting mechanical actuation may be an irreversible process.

The term flexible structure should be construed broadly, and should be construed to at least include a cantilever (or flapper)-type structure, a bridge structure, where a beam is clamped in two ends, as well as to include a diaphragm or membrane structure attached to a platform along parts of, or the entire, periphery.

Examples of detector devices include situations where the indicator substance in addition to being coloured or colour inducing may also be detectable by an additional means. The indicator substance may also be magnetic, in such a situation may the readout also be performed by means of a device capable of detecting magnetism. The indicator substance may also be radioactive, and the readout may also be performed by means of a device capable of detecting radioactive radiation, the indicator substance may also emit UV-light or infrared light, and the readout may also be performed by means of a device capable of detecting UV-light or infrared light, etc.

The actuation of the flexible structure may further be conditioned upon a physical trigger. The interaction occurring between the flexible structure (or possibly a functionalization layer or species on the flexible structure) and the target substance may be conditioned upon the presence of a given condition e.g. a chemical reaction between the target substance and the flexible structure (or possibly functionalization layer or species on the flexible structure) may be condition upon a given temperature, a given pressure, the presence of radiation, such as visible or near visible light, UV light and/or infrared light. Other types of physical triggers may be envisioned.

The energy stored in the flexible structure may be released by inducing a change in surface stress of the flexible structure. A change in surface stress should also be construed to include change in interfacial stress between a surface of the flexible structure and one or more layers forming an interface with the flexible structure. The change in surface stress of the flexible structure may e.g. be obtained from an etching of a coating layer, from a deposition of a substance, from a change in electrostatic and/or steric interactions between species on the functionalized surface upon exposure of the target substance, from a change in entropy upon exposure to the target substance, from a change in osmotic pressure between the species on the functionalized surface upon exposure of he target substance, etc.

The functionalization of the flexible structure may be provided by the coating layer, or by one or more coating layers. The one or more layers may comprise a metal layer and/or a molecular layer and/or a salt layer, the metal layer may be a first layer, whereas the molecular layer and/or salt the layer may be a first or a second layer, the second layer being deposited onto the first layer.

One or more of the one or more coating layers may thus both provide, or be part of, a functionalization of the flexible structure, as well as provide a surface or interfacial stress change originating from a mismatch between the coating layer and the flexible structure.

The coating layer may more specifically be a layer of Au, Ag, Pt, Pd, Al, Cr, Ti, Cu, Ru, Rh, or any combination or alloying of such or other metals. The layer may be provided by vapour deposition, precipitation, laser ablation, electron beam evaporation, electro-plating, shadow masking, or any other suitable technique for providing a metal layer. The coating layer may have a thickness between a few nanometres to a few hundreds nanometres.

An outer surface of a flexible structure may be surface treated prior to, or as an alternative to, providing the first layer and/or the second layer. For example, an outer surface may be roughened. A rough surface may promote attachment of chemical substances to the surface, such as promote immobilization of molecular species directly on the polymer.

The coating layer may also be a molecular layer, such as a layer of receptor molecules, capable of selectively binding specific molecules, such as macro-molecules, bio-molecules, DNA, such as single or double stranded DNA e.g. from disease-associated genes, antigens, nucleic acids, amino acids, proteins, cells, such as cardiac cells, bacteria, virus, fungus, micro tubili, various drug molecules, constituents of explosives, e.g. TNT, traces of toxins, warfare agent, polymers, etc. The coating layer may be a self-assembled layer, such as a self-assembled monolayer or multilayer.

The flexible structure may prior to coating the first surface be pre-loaded, e.g. by coating the second surface of the flexible structure with a metal layer, such as Au, Ag, Pt, Pd, Al, Cr, Ti, Cu, Ru, Rh, or any combination or alloying of such or other metals. The pre-loading may be obtained from an induced surface or interfacial stress from the coating of the second surface, alternatively may the pre-loading be due to an internal stress of the flexible structure causing it to bend. The coating of the first surface may be such that the induced surface stress from this coating layer counter-balance the induced surface stress of the coating layer on the second surface, or alternatively counter-balance an internal stress. Furthermore, any coating layers, such as metal layers deposited on the first or second surface may be coated with a protection layer, such as an anticorrosive lipid layer, thereby avoiding undesirable chemical reactions between a fluid and the coating layer. Such protection layers may e.g. be removed by a chemical reaction resulting from a substance added to the target substance, or possibly by a chemical reaction with the target substance itself.

The first surface and/or second surface may additionally or alternatively be coated with nano-size particles or micro-size particles, such as particles of: Au, polystyrene, SiO₂.

Thus, the first surface and/or second surface may be coated with a single layer or a multilayer, such as a multi-metal layer or mixed layers.

The deflection of the flexible structure may be in the range of 100 nm to 1 mm, such as in the range of 500 nm to 0.5 mm, such as in the range of 1 µm to 0.1 mm. It is important that the deflection of the flexible structure is large enough to release detectable amounts of indicator substance. For a cantilever type flexible structure may the deflection be taken as the maximum deflection of one the free end with respect to the clamped end. For a bridge structure or a membrane structure, may the deflection be taken as the deflection of a central part, or other deflected part, with respect to a periphery (or end) point.

The flexible structure may be a polymer-based component, i.e. may be fabricated in a polymer-based material. The polymer-based material may be any suitable type of natural or synthetic polymer-based material or co-polymer-based material which may sustain a stable structure in the micrometer or sub-micrometer range. The polymer-based material may be a plastic material, such as a thermoplastic or a thermoset, or such as a so-called photoplastic, i.e. a plastic material that may be photolithographically processed. The material of the polymer-based flexible structure may be selected from the group consisting of: SU-8 based polymers, such as XP SU-8 polymer, polyimides or BCB cyclotene polymers, parylene, polystyrene, cyclic olefin copolymer (COC) polymers polymethylmetacrylate (PMMA) and polydimethylsiloxane (PDMS), but many other polymer-based materials or plastic materials could be used. The chemical name of SU-8 is glycidyl ether of bisphenol A. SU-8 may be a suitable component for fabricating at least part of a sensor system since it has a high chemical resistance, it is compatible with conventional microfabrication techniques, capable of supporting very high aspect ratios and it is relatively easy and fast to process.

It is an advantage to use a polymer-based material as the material of the flexible structure, since compared to a silicon-based cantilever a more sensitive sensor system may be provided. Furthermore, a high interfacial stress change may occur between a polymer and a deposited metal layer, thereby facilitating a large deflection of the flexible structure. Nevertheless, at least for some applications may a non-polymer based material, such as Si, be used.

The material of the polymer-based flexible structure and the at least first fluid system may be made from the same materials, i.e. those materials as already mentioned.

A further advantage is that by use of a polymer material, the fabrication process is rendered simple, cheap, fast and flexible. It is an advantage that a cheap device may be provided, e.g. since a sensor system may be used to measure chemical and/or biological substances which may be difficult to, time consuming to or even toxic to clean off a device, rendering it desirable to provide a single-use and possibly disposable device.

The second fluid system may further comprise a coloured surface which is exposed after actuation of the flexible structure, such as a coloured surface placed in the bottom of the container. The presence of a coloured surface may enhance a detection by the human eye.

The second fluid system may contain different types of indicator substances. In an embodiment may the second fluid system contains a dry or liquid colour inducing substance, which is dissolved by the target substance or by a chemical added to the target substance after actuation of the flexible structure. In another embodiment may the second fluid system contain a dry of liquid indicator substance, which reacts with the target substance or by a chemical added to the target substance after actuation of the flexible structure, and where the reaction product is detected. The release of the indication substance may result in a sedimentation of a coloured layer and/or coloured particles in the first fluid system as well as possibly in the second fluid system. It may result in a colouring of a substance. The colouring of a substance may e.g. be obtained by fluorescence process, by chemiluminescent process, by a colouring of the target substance, by a coloured reaction product, by providing amplitude or fluorescent contrast to the target substance.

The first fluid system may further comprise a filter upstream of the flexible structure, the filter affecting the flow of the target substance and/or any released fluid from the second fluid system.

The sensor system may comprise an array of flexible structures, such as a parallel or a serial array of flexible structures. Each flexible structure may be coupled to a separate or a shared second fluid system. The first fluid system may comprise channels, and an array of flexible structures may be provided so that each channel contain one flexible structure separating the channel from the second fluid system, or an array of flexible structures may be provided so that each channel contain an array of flexible structure separating the channel from the second fluid system. Furthermore, at least two of the flexible structures in the array may be provided with different mechanical properties and/or chemical properties.

The provision of an array may provide a sensor system capable of sensing a multitude of different target substances, or may be a way of releasing a larger amount of indicator substance, that may be practically to release from a single container, or second fluid system.

The indicator substance may be an ink, such as a nutrition colour and the target substance may be an acid, such as NaOH.

In general may the indicator substance also be or contain a nutrition/water soluble colour, an oil, grease or wax, carbon particles, fluorescent dyes, such as rhodamine, fluorescein, etc., colloids of polystyrene, SiO₂, Au, Ag, particles that are be functionalized with e.g. fluorescent dyes and other biomolecules, oxidants, enzymes, pH-sensitive dyes, etc.

In general may the target substance also be or contain: HF, silanes, peroxides, alcohols, pesticides, proteins, enzymes, DNA, cells, fungus, bacteria, virus, glucose, explosives, etc.

In an embodiment the indicator substance is contained in a reservoir separate from the second fluid system. In such an embodiment release of the indicator substance into the second fluid system may be done by a user before use, thereby avoid unintended release e.g. during transportation.

In an embodiment may the flexible structure be actuated by means of an induced strain. For example from a bi-morph effect relating from a temperature dependent expansion coefficient of different layers of the flexible structure.

According to another aspect of the present invention, a method of sensing the presence of at least one target substance in a fluid, the target substance being provided in an at least first fluid system, the first fluid system being separated from a second fluid system by a flexible structure,
wherein the second fluid system containing an indicator substance, the indicator substance being a coloured substance or a colour inducing substance, the flexible structure having a first surface facing the at least first fluid system, the first surface being functionalized to interact with the at least one target substance in a fluid present in the at least first fluid system, the flexible structure having a second surface facing the second fluid system, and
wherein the flexible structure upon exposure to the target substance releases energy stored in the flexible structure resulting in a mechanical actuation of the flexible structure, the mechanical actuation of the flexible structure resulting in a release of the coloured indictor substance either into the first fluid system or into a third fluid system connected to the second fluid system, or resulting in a colouring of a substance released either into the first fluid system or into a third fluid system connected to the second fluid system.

According to a further aspect of the present invention is a process for manufacturing the sensor system according to the first aspect of the invention provided. The process comprising the steps of
- providing a layer of a first material on a first substrate,
- masking the first material to indicate a pattern,
- defining the pattern by way of etching or developing to form at least one flexible structure and at least a first fluid system, the flexible structure having a first and a second surface, thereby forming a fluidic system and
- providing a second substrate of a second material,
- providing at least a second fluid system in the second substrate, and
combining the fluidic system and second substrate.

The process may further comprise the step of providing a third fluid system in the second substrate. The process may also include the provision of other structures in the first system, such as fluid channels, walls, inlet(s), outlet(s), etc.

Also a lid may be provided to the fluidic system, and optionally may an intermediate layer be provided between the fluidic system and the lid, as well as between the fluidic system and the second substrate. The intermediate layer may be a layer of flexible polydimethylsiloxane (PDMS).

The first material may be a radiation definable polymer, where the process further comprises the steps of
- patterning the radiation definable polymer through exposing to radiation,
- developing the patterned radiation definable polymer to form at least one flexible structure and at least a first fluid system, thereby forming a fluidic system.

The radiation definable polymer may be exposed using e-beam, I-line, X-rays or near-UV lithography.

A radiation definable polymer is a polymer which in the unexposed state is sensitive to radiation so that the radiation definable polymer may be defined, that is in a specific form or shape, by irradiating parts of the polymer while leaving other parts unexposed. During exposure or by further processing, such as by heating or baking, either the exposed or the unexposed parts (positive or negative process) become cross-linked where after the cross-linked polymer is no longer radiation definable and thus no longer radiation sensitive.

It is an advantage to being able to use standard photolithography, since it may reduce the complexity of the fabrication process and thus further reduces the cost of the final sample. And since the costs of e.g. single use samples need to be reduced in order to allow for widespread use of such single use devices, reduction of the manufacturing costs are an essential need for the continued growth of the market for such devices.

The first substrate may be of glass, polymer, ceramic, plastic, preferably a semiconductor, such as GaAs, GaInAsP, preferably such as silicon. The second substrate may be polymethylmetacrylate (PMMA) or polydimethylsiloxane (PDMS) substrate.

In an alternative process for manufacturing the sensor system, the process is a moulding technique wherein the first material is provided into a mould so that at least part of the at least first fluid system is formed in one piece.

The moulding process may also include providing the second material into a mould so that at least part of the at least second fluid system and/or the at least third fluid system are formed in one piece.

The moulding technique may be an injection moulding technique or an embossing moulding technique. The moulding may be a sequential moulding process. A sacriflcial layer, or anti-sticktion layer may be provided to the mould so as to facilitate release of the moulded system. The moulding process may include injection into the mould at high pressure, and possibly at elevated temperature. The mould may be fabricated in Silicon, in a metal, in an oxide or in any other suitable material.

In another alternative process for manufacturing the sensor system, the process is a laminating technique where two or more films are laminated together to form a single structure. The laminating may be combined with inserting a layer, a chip or other circuit, etc. between two laminated layers.

A combination of techniques, such as a combination of the above or other techniques may be applied. In general may the sensor be fabricated using additional processes known from the semiconductor industry, such processes including spin-coating, baking, wet- and dry etching, physical- and chemical vapour deposition, doping, etc. Also such fabrication steps as: imprinting, laser machining and micromilling, etc may be employed.

The invention is described by the description of a sensor system a method of sensing the presence of at least one target substance in a fluid and a process for manufacturing a sensor system, and it is envisaged, that features described in relation to one aspect only, may also be applied to other aspects of the invention as herein described.

### BRIEF DESCRIPTION OF THE DRAWINGS

Embodiments of the invention will be described, by way of example only, with reference to the drawings, in which
FIG. 1 illustrates an embodiment of the actuation principle of the present invention,
FIG. 2 illustrates an embodiment of a sensor,
FIG. 3 illustrates an embodiment where the fiexible structure is actuated upon a change in surface stress,
FIG. 4 illustrates a test experiment to show the performance of a sensor,
FIG. 5 illustrates a complete senor chip for test purpose,
FIG. 6 illustrates in cross-sectional view an alternative embodiment of a sensor,
FIG. 7 illustrates an optical microscopy image of a capillary gauge used for proof of principle,
FIG. 8 illustrates different embodiments of capillary gauge configurations
FIG. 9 illustrates another embodiment, than the embodiment of FIG. 3 where the flexible structure is actuated upon a change in surface stress,
FIG. 10 illustrates an embodiment where the first fluid system further comprising a filter,
FIG. 11 illustrates an embodiment resulting in a sedimentation of released particles,
FIG. 12 illustrates an embodiment where the second fluid system further comprises a coloured surface,
FIG. 13 illustrates a situation where the indicator substance reacts with a substance added to the target substance,
FIG. 14 illustrates an embodiment where the readout is assisted by a waveguide,
FIG. 15 illustrates a first embodiment of an array of flexible structures,
FIG. 16 illustrates a second embodiment of an array of flexible structures,
FIG. 17 illustrates an alternative embodiment of a flexible structure.

### DESCRIPTION OF EMBODIMENTS

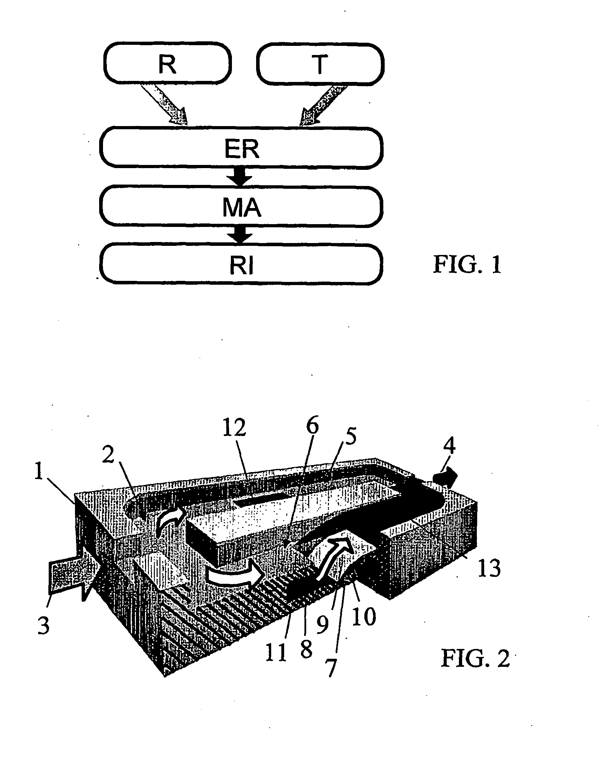

FIG. 1 illustrates an embodiment of the actuation principle of the present invention. A receptor R bound on the surface of the flexible structure is exposed to a target substance T resulting in a chemical reaction on the surface of the flexible structure, which further results in an energy release ER of energy stored in the flexible structure, that subsequently results in a mechanical actuation MA of the flexible structure, and further in a release RI of a coloured or colour inducing indicator substance. A self-actuated flexible structure capable of operating without power supplied from an external source may thereby be provided, and where it is possible to detect a readout by the human eye. In a situation where the flexible structure is not exposed to a target substance, the flexible structure is not actuated, facilitating an array for detecting multiple target substances.

FIG. 2 illustrates an embodiment of a sensor applying the actuation principle of FIG. 1 for sensing the presence of a target substance in a fluid, and for providing a visual indication of a presence of the target substance in the fluid.

The sensor system 1 comprising a first fluid system 2 for directing a fluid from an inlet 3 to and outlet 4, the inlet and outlet may further be connected to an external fluid system or other means of providing a fluid. The first fluid system in the illustrated embodiment has two fluid channels 5, 6, each fluid channel comprising a flexible structure 7, 12 separating the first fluid system and a second fluid system 8, the flexible structure being a square flapper, or cantilever-type structure. The second fluid system being in this embodiment a closed container holding an indicator substance, such as a coloured ink 11. The flexible structure has a first surface 9 facing the first fluid system, and a second surface 10 facing the second fluid system.

In the situation the flexible structure is exposed to a target substance, energy stored in the flexible structure is released resulting in a mechanical actuation of the flexible structure. The flexible structure denoted 7 is illustrated to be open, but initially the structure was closed, and upon actuation, i.e. opening of the flapper, the indicator substance contained in the second fluid system is released into the first fluid system. In one embodiment the indicator substance is a colour ink, so that upon detection of the presence of the target substance, the result is a colour change 13 of the fluid in the fluid channel after the flexible structure, the colour change may the be detected, e.g. with the human eye. In the Figure, it is also illustrated, that even though both flexible structures 7, 12 are interacting with the fluid, the second flexible structure 12 remains closed, i.e. this flexible structure 12 is functionalized for another target substance.

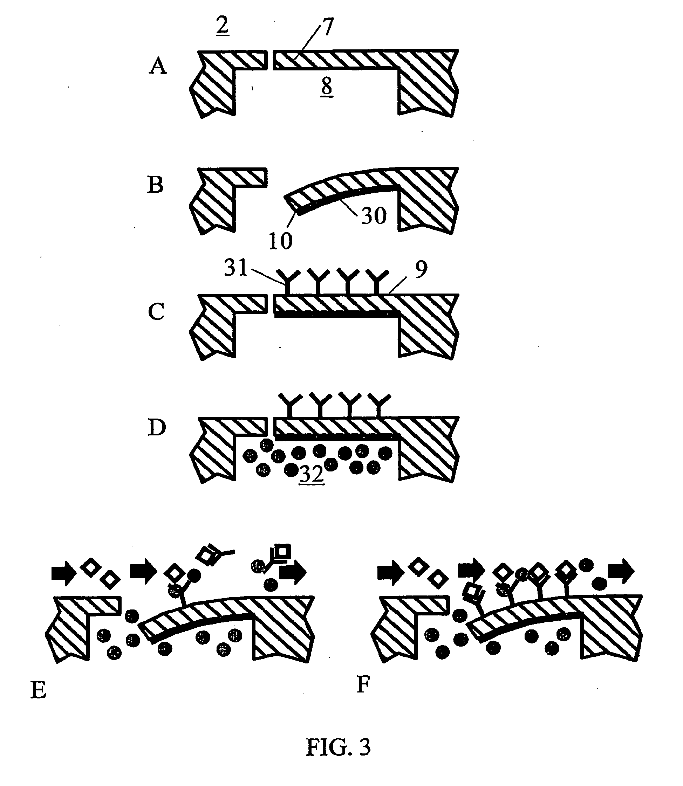

FIG. 3 illustrates a schematic embodiment of the present invention where the flexible structure is actuated upon a change in surface stress as induced by a reaction between an analyte containing the target substance and a coating layer of the flexible structure.

FIG. 3A illustrates the flexible structure 7 in an initial form, the flexible structure separating a first fluid system 2 and a second fluid system 8. In FIG. 3B is the flexible structure pre-loaded by coating the second surface 10, i.e. the surface facing the second fluid system with a coating layer 30, the coating layer being a stressed layer resulting in a downward (or inward) deflection of the flexible structure. In FIG. 3C the first surface 9 of the flexible structure is functionalized by coating with a reactive layer 31, the reactive layer balancing the surface stress induced from coating the second surface. The reactive layer is shown as a layer of separate receptor species, this is for illustrative purposes only. The reactive layer may be any type of layer within the scope of the invention. The steps 3B and 3C may be performed in reverse order. In FIG. 3D the container is filled with the indicator substance 32. FIG. 3E and 3F illustrate two embodiments of detection of a target substance in an analyte. In FIG. 3E the target substance reacts with the reactive layer in a way so that the reactive layer is removed from the flexible structure resulting in that the flexible structure bends, i.e. flips back to the configuration of FIG. 3B. In FIG. 3F the target substance reacts with the reactive layer in a way so the reaction product stays on the surface of the flexible structure, the reaction product having a smaller (or larger) surface stress resulting in that the flexible structure flips inward (or outwards). In a situation as in FIG. 3F may the step of FIG. 3B be avoided, since the actuation may arise form the surface stress change induced by the reaction product. The process step of FIG. 3B is consequently optional.

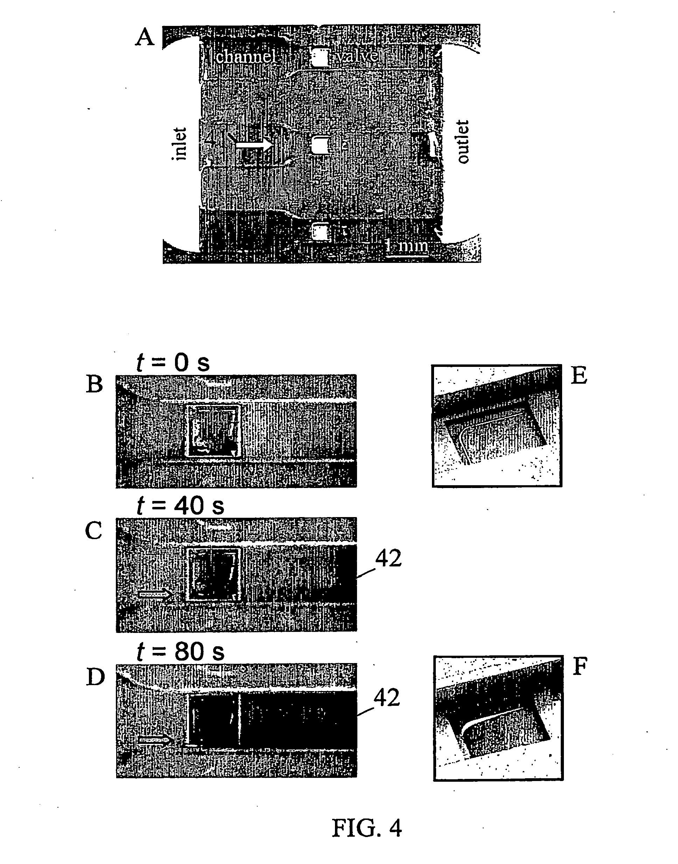

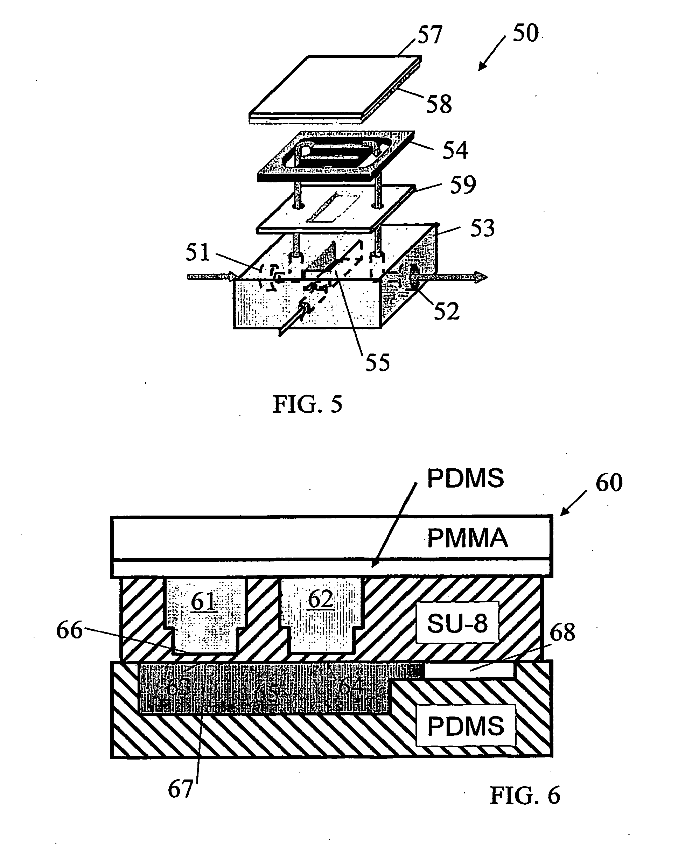

FIG. 4 illustrates a test experiment to show the performance of a sensor. The sensor system is basically fabricated as illustrated in FIGS. 2 and 3. The actual test sensor is further described in connection with FIG. 5.

In this embodiment, the flexible structure is surface stress driven by coating a polymer flexible structure with a metal layer on each surface and thereby providing a flexible structure which reacts with acids and lyes. The actuation employs elastic deformation of the flexible structure provoked by stress in the evaporation-deposited metal coating. The stress can either be caused extrinsically by a mismatch in the thermal coefficient of expansion of the metal film and the substrate or intrinsically during the film nucleation. Since the metal deposition here is performed at room temperature thermal stress can be neglected. The stress in the metal film is dominated by intrinsic sources such as material defects. For normal temperatures as here the stress in the metal film is typically tensile.

A fully self-actuated valve, i.e. a combination of a flexible structure and a container, is achieved by the following procedure. The flexible structure is first coated with a thin Ti layer 30 on the bottom side as shown in FIG. 3B. The Ti bends the flexible structure down due to the intrinsic tensile stress. This deformation creates a gap between the flexible structure and the channel, which corresponds to a valve in an opened state. The flexible structure is straightened by evaporating an Al layer 31 on the top surface 9 to balance the stress induced by the Ti layer (FIG. 3C). In FIG. 3C, the reactive layer is shown as a layer of separate receptor species, this is for illustrative purposes only, a Ti layer would not be in this form, but in the form of a film or layer similar to the Al layer on the top surface. The deposited Al layer exhibits a counteracting intrinsic tensile stress. In this state, the flexible structure is ready for implementation. Upon selective etch of the Al film by, e.g., an NaOH solution as shown In FIG. 3E the tensile stress on the top of the valve is released bending the flexible structure back to the initial open position. By this, the flexible structure releases a solution stored in the container (the second fluid system 8) below the flexible structure into the channel above (the first fluid system 2). The flexible structure is fabricated in SU-8.

Fig. 4A shows a top view of an SU-8 chip containing an array of three flexible structures fed by individual microfluidic channels, the image is an optical light microscope image. The valves and channels were fabricated by stacking and structuring several layers of SU-8 on a fluorocarbon-coated Si wafer. The SU-8 was structured using standard UV lithography. Soft- and post-exposure baking of the resin was performed at 40 °C to reduce thermally Induced stress. After the microfabrication of the SU-8 was completed, the individual chips were simply peeled off the Si substrate.

The metal films were evaporation-deposited onto the flexible structure after the release of the chip by the use of shadow masks. FIG. 4E shows scanning electron microscope images of a closed SU-8 flexible structure balanced by 160 nm Ti on the bottom side (deposition rate: 10 Å/s) and 400 nm Al on the top side (deposition rate 2 Å/s) as illustrated schematically in FIG. 3C (again with Ti in the form of a film or layer). The flexible structure measures 400 µm x 400 µm and is 8 µm thick. Proper levelling of the flexible structure to the channel floor resulted in a minimum residual gap between the two of about 4 µm.

FIG. 4B to 4D show optical microscope images of releasing green ink from a reservoir below the flexible structure upon introduction of a 0.5 M NaOH solution at the time t = 0 (FIG. 4B). The arrow 41 in FIG. 4A indicates the flow direction. The Al film on the flexible structure is continuously etched and delaminating from the surface Increasing the deflection and thus the throughput of the ink from the reservoir. The ink is visible 42 as the dark liquid downstream of the flexible structure. FIGS. 4B to 4D shows the effective release of the green marker upon contact of the flexible structure surface with the NaOH solution, the ink is starting to be released already at t = 40 s (FIG. 4C), whereas at t = 80 s (FIG. 4C), the ink is released.

The reservoir below the valve array was filled with a green, water-soluble marker (nutrition colour). The microfluidic network In the SU-8 above the flexible structures was first filled with water by a syringe pump and rinsed for about 5 min. Subsequently, an analyte composed of 0.5 M NaOH was introduced. The velocity of the solution over the flexible structure amounted to about 5 mm/s. The initial leakage through the residual gap between the flexible structure and the channel bottom proved to be negligible. The NaOH solution selectively etched the Al film, which resulted in a decrease in the layer thickness and regularly observed delamination of the film from the SU-8 substrate. Both mechanisms reduced the tensile stress on the top side of the flexible structure. The Ti layer on the bottom side remained Intact during the whole experiment, pulling the flexible structure down. The flexible structure depicted in FIG. 4F was imaged after the testing. The downward bending of the flexible structure is clearly visible providing evidence that the release of the marker was indeed due to a self-actuated opening of the flexible structure. The maximum deflection of the flexible structure end depicted in FIG. 4F measures 45 µm. Here, the deflection is defined as the distance between the channel floor and the top front edge of the flexible structure. Flexible structures exposed to pure water for a time similar to the duration of the whole experiment using NaOH showed no visible deterioration of the Al film. The flexible structure remained closed providing evidence of a specific lye-induced self-actuation of the flexible structure in contact with NaOH.

A complete senor chip 50 for test purpose is illustrated in FIG. 5. The inlet 51 and outlet 52 feeds for the fluidic network in the SU-8 chip 54 were fabricated In a 10 mm thick polymethylmetacrylate (PMMA) support 53 by mechanical drilling. The feeds were equipped with threads to connect the system via PFTE tubing to a pump and reservoirs for metal etchants and water. The same PMMA support 53 also host the second fluidic system 55 comprised of a reservoir with inlet and outlet holes. The reservoir 55 supplies the complete valve array and is fabricated by infrared laser drilling. The top of the fluid system 54 is covered by a glass lid 57 with a thin layer 58 of flexible polydimethylsiloxane (PDMS) for sealing. Another infrared-laser-machined PDMS sheet 59 was inserted as a sealing between the SU-8 system 54 and the PMMA support. The individual parts is clamped together by an aluminium lid screwed onto an aluminium base part (not shown). This construction allows for unobstructed observation of the flexible structure array from above using an optical microscope.

FIG. 6 illustrates in cross-sectional view an embodiment of a sensor different from the sensor illustrated in FIG. 2, however the sensor of FIG. 6 also applying the actuation principle of FIG. 1 for sensing the presence of a target substance in a fluid, and for providing a visual indication of a presence of the target substance in the fluid.

The sensor system 60 comprising a first fluid system 61, 62 being constituted of two fluid channels for directing a fluid from an inlet to and outlet, the inlet and outlet are not shown. Each fluid channel of the first fluid system comprises a flexible structure 63, 64 separating the first fluid system and a second fluid system 65. The second fluid system being a closed container holding an Indicator substance, such as a coloured ink. The flexible structure 63, 64 has a first surface 66 facing the first fluid system, and a second surface 67 facing the second fluid system.

An important difference as compared to the embodiment of FIG. 2 is that the flexible structure is not a flapper, but a membrane. The flexible structure therefore does not open or close upon exposure to a target substance, but bends or presses downwards or upwards.

The flexible structure is illustrated to be in a balanced configuration, but upon actuation the membrane exerts a pressure on the indicator substance contained in the second fluid system and thereby releases the indicator substance into a third fluid system 68 connected to the second fluid system.

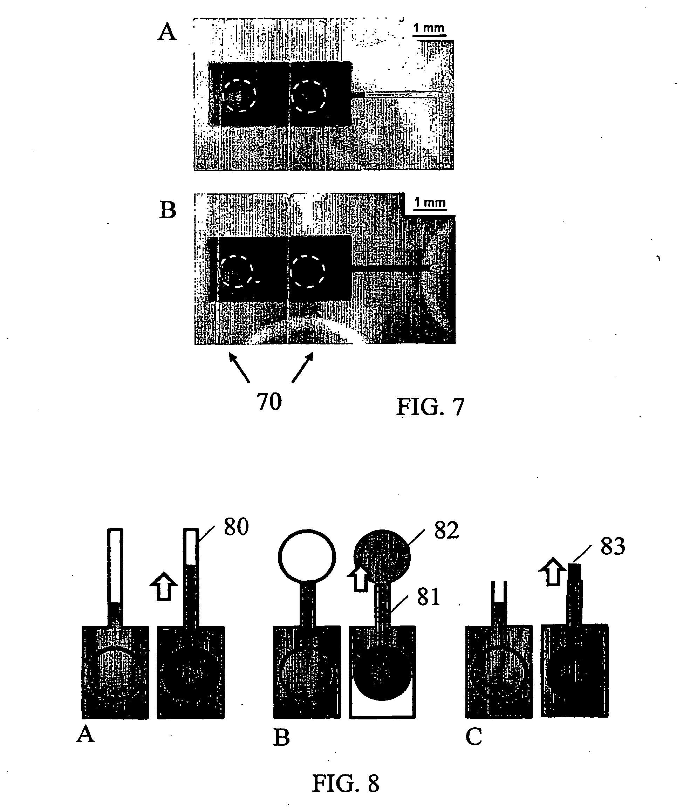

FIG. 7 illustrates an optical microscopy image of a capillary gauge, with capillary length of 3 mm, a width of 200 µm, and a depth of 50 µm. The colour is a green nutrition colour. The Figure illustrates a proof of principle, where water is pressed through the channels 70 at a low pressure in FIG. 7A, and a higher pressure in FIG. 7B. It is seen that the water presses the membranes down, so that the indicator substance is released into the third fluid system 71.

FIGS. 8A to 8C illustrate different embodiments of capillary gauge configurations, where each Figure illustrates a situation before the flexible structure is actuated (left side) and a situation after the flexible structure has been actuated (right side). In FIG. 8A the indicator tube, i.e. the third fluid system 80 is a closed capillary tube. The tube may be straight or curved (e.g. spiralled). In FIG. 8B the indicator tube 81 is also a closed capillary tube, but where a larger compartment 82 is filled upon actuation of the flexible structure. In FIG. 8C the indicator tube is an open capillary. In this embodiment the actuation of the flexible structure induces release of liquid 83, e.g. into a fluidic system, such as a microfluidic system, with similar read-out setups as for the flapper.

The capillary inside of the tubes may be repared so as to adjust the wettability between the liquid and reservoir to achieve repulsion between the two. For an aqueous liquid may the container be or be rendered hydrophobic. For a lipid solution may the container be or be rendered hydrophilic.

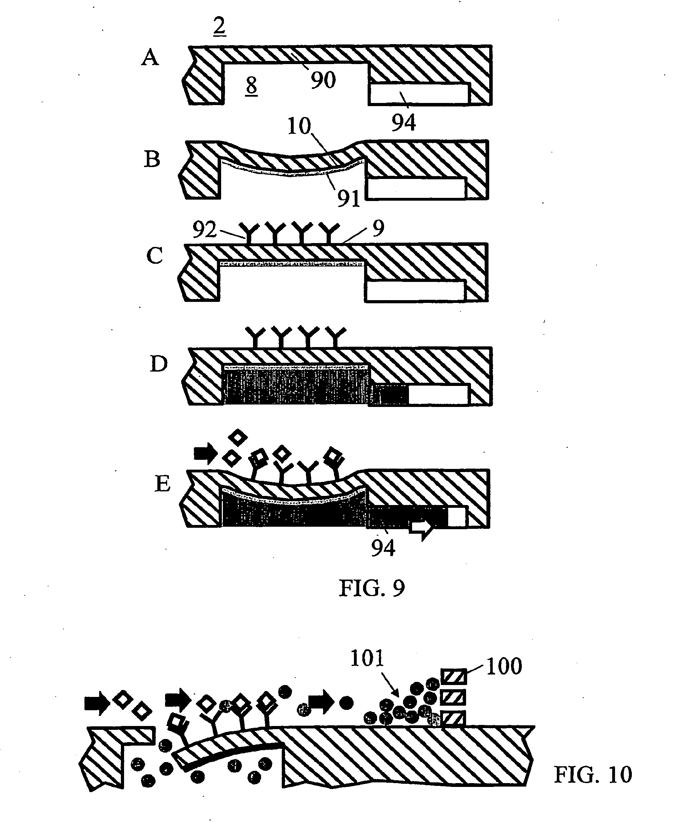

FIG. 9 illustrates an embodiment of the present invention similar to FIG. 3, except that where the flexible structure is a membrane structure as discussed in connection with FIGS. 6 to 8.

FIG. 9A illustrates the flexible structure 90 in an initial form, the flexible structure separating a first fluid system 2 and a second fluid system 8, the second fluid system being connected to a third fluid system 94. In FIG. 9B is the flexible structure pre-loaded by coating the second surface 10, i.e. the surface facing the second fluid system with a coating layer 91, the coating layer being a stressed layer resulting in a downward (or inward) deflection or bending of the flexible structure. In FIG. 9C is the first surface 9 of the flexible structure functionalized or coated with a reactive layer 92, the reactive layer balancing the surface stress induced from coating the second surface The reactive layer 92 is shown as a layer of separate receptor species, this is for illustrative purposes only. The reactive layer may be any type of layer within the scope of the invention. In FIG. 9D is the container filled with the indicator substance 93. In FIG. 9E the target substance reacts with the reactive layer in a way so that the reaction product having a smaller (or larger) surface stress resulting in that the flexible structure flips inward (or outwards), there by releasing, i.e. pressing, the indicator substance into the third fluid system 94. As in connection with FIG. 3B, may the pre-loaded step of FIG. 9B be optional, and the steps of FIG. 9B and 9C may be performed in reverse order. In a situation where the flexible structure flips outwards, may the third fluid system be at least partly filled.

FIG. 10 illustrates an embodiment where the first fluid system further comprising a filter 100 upstream of the flexible structure, the filter affecting the flow of the target substance and the released fluid from the second fluid system, so that an aggregation 101 of released particles is obtained by the filter. A readout may then be performed from a colouring of the area adjacent to the filter.

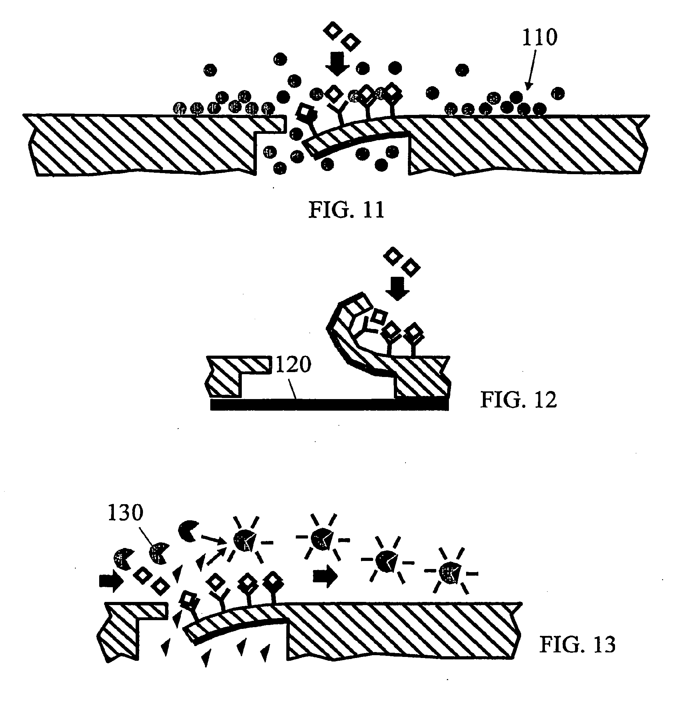

FIG. 11 illustrates an embodiment where the second fluid system contains an indicator substance which after release from the second fluid system results in a sedimentation 110 of the released particles. The sedimentation may be a coloured sedimentation which may be detected from a colouring of the area surrounding the flexible structure.

FIG. 12 illustrates an embodiment where the second fluid system further comprises a coloured surface 120 which is exposed after actuation of the flexible structure.

FIG. 13 illustrates a situation where the indicator substance stored in the second fluid system reacts with substance 130 added to the target substance. The indicator substance may e.g. be a dissolved or dry colour substance, a dry colour substance will be dissolved when the flexibte structure opens, providing amplitude, fluorescent contrast or chemiluminescent contrast which may be read out. Dried indicator substance may e.g. be metal particles, metal oxide particles or polystyrene particles.

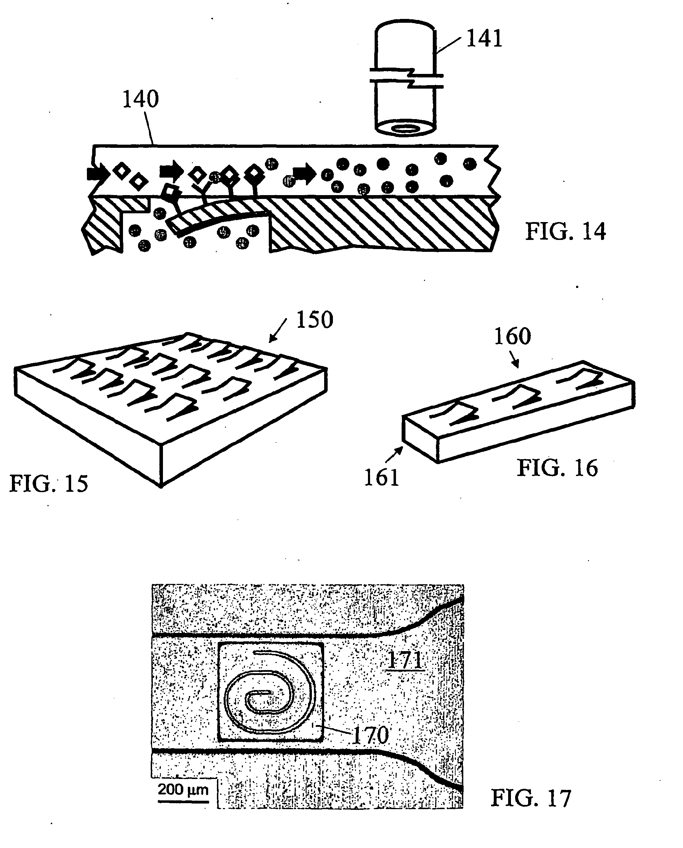

FIG. 14 illustrates an embodiment where the readout is assisted by a waveguide 141. This may e.g. be useful for conducting measurements in a compartment where direct visible contact with the sensor system is not feasible. In this embodiment the fluid channel is closed with a lid 140, so that the sensor system may safely be inserted into the compartment without risk of leaking the target substance, the indicator substance or any other substances present in the fluid system. The waveguide is placed in a normal configuration, however the waveguide may also be tilted, or it may be incorporated in to the fluidic system itself, i.e. be in-plane with the fluid channel, and fabricated as a part of the sensor system. The waveguide may be connected to or replaced by a detector, such as a CCD detector or other type of detector.

An array of flexible structures may be provided in several ways. FIG 4A illustrates an array where each flexible structure is connected to a separate fluid channel. FIGS. 15 and 16 illustrate alternative embodiments of arrays of flexible structures. FIG. 15 illustrates an array 150 of flexible structures coupled to a single fluid channel. The array of the flexible structures may either share a container, i.e. the second fluid system, filled with indicator fluid, or separate containers for indicator fluids may be applied.

FIG. 16 illustrates a serial array 160, where fluid is passed over the flexible structures from one side 161. Again may the flexible structures either share a container or separate containers for indicator fluids may be applied.

FIG. 17 illustrates a light microscope image of an alternative embodiment of a flexible structure 170 in a fluid channel 171. The flexible structure is prepared as a spiral (other shapes may be envisioned). It may be advantageous to provide a spiral since larger deflection on a smaller area may be provided, since the length of the flexible structure is increased on a given area, thereby providing a more efficient release.

The present invention may be used in a large variety of applications. Possible applications include: Monitor devices, e.g. a food monitor from production to a consumer, e.g. during shipping and storage, e.g. to counter food abuse, during production e.g. to detected the presence of bacteria at an early stage. A sensor may e.g. monitor that the temperature of a product has been kept lower than a specified temperature or a specified humidity, that certain scents are not present, e.g. scents indicating a putrefaction, that a product has not been exposed to radiation, etc. Since the present invention does not require a power source and further may be readout directly with the human eye, e.g. without use of computer or other equipment, the present invention may be used for sensors to be applied in third world countries for monitoring water quality, etc. In other applications may the invention be used for diagnosis purposes, such as point-of-care diagnosis, screening for the presence of various pathogenic substances or indicators. In other applications may the sensor be used in an alarm system triggered by a specified concentration or presence of a poisonous gas or liquid. The sensor may be used as a sensor of biological and chemical warfare agents, as a sensor of environmental toxins, etc.

It is to be understood that the application of the present invention is not limited to the above-listed examples.

Although the present invention has been described in connection with preferred embodiments, it is not intended to be limited to the specific form set forth herein. Rather, the scope of the present invention is limited only by the accompanying claims.

## Claims

1. Sensor system (1, 60) comprising:
at least a first fluid system (2, 61, 62),
a second fluid system (8, 65) containing an indicator substance (11, 32, 93), the indicator substance being a coloured substance or a colour inducing substance,
a flexible structure (7, 12, 63, 64, 90) separating the at least first fluid system and the second fluid system, the flexible structure having a first surface (9, 66) facing the at least first fluid system, the first surface (9, 66) being functionalized to interact with at least one target substance (T) in a fluid present in the at least first fluid system, the flexible structure having a second surface (10, 67) facing the second fluid system,
wherein the flexible structure upon exposure to the target substance releases energy (ER) stored in the flexible structure resulting in a mechanical actuation (MA) of the flexible structure, the mechanical actuation of the flexible structure resulting in a release (RI) of the coloured indicator substance either into the first fluid system or into a third fluid system (68, 71, 80, 94) connected to the second fluid system, or resulting in a colouring of a substance released either into the first fluid system or into a third fluid system connected to the second fluid system.

2. Sensor system (1, 60) according to claim 1, wherein the actuation of the flexible structure (7, 12, 63, 64, 90) is further conditioned upon a physical trigger.

3. Sensor system (1, 60) according to any of the preceding claims, wherein the physical trigger is a predetermined temperature of the target substance (T), is predetermined pressure of the target substance and/or is a radiation incident upon the target substance and/or the flexible structure (7, 12, 63, 64, 90).

4. Sensor system (1, 60) according to any of the preceding claims, wherein the energy stored in the flexible structure (7, 12, 63, 64, 90) is released by inducing a change in surface stress of the flexible structure.

5. Sensor system (1, 60) according to claim 4, wherein the change in surface stress of the flexible structure (7, 12, 63, 64, 90) is obtained from an etching of one or more coating layers (30). '

6. Sensor system (1, 60) according to any of the preceding claims, wherein the first surface (9, 66) is coated with one or more metal layers, one or more molecular layers and/or one or more salt layers.

7. Sensor system (1, 60) according to claim 6, wherein prior to coating the first surface (9, 66), the flexible structure (7, 12, 63, 64, 90) is pre-loaded.

8. Sensor system (1, 60) according to any of the preceding claims, wherein the second surface (10, 67) is coated with one or more metal layers, one or more molecular layers and/or one or more salt layers.

9. Sensor system (1, 60) according to claim 6, wherein the one or more metal layers is coated with a protection layer.

10. Sensor system (1, 60) according to any of the preceding claims, wherein the first surface (9, 66) and/or second surface (10, 67) is/are coated with nano-size particles or micro-size particles.

11. Sensor system (1, 60) according to any of the preceding claims, wherein the deflection of the flexible structure (7, 12, 63, 64, 90) is in the range of 100 nm to 1 mm.

12. Sensor system (1, 60) according to any of the preceding claims, wherein the flexible structure (7, 12, 63, 64, 90) is polymer-based.

13. Sensor system (1, 60) according to any of the preceding claims, wherein the second fluid system (8, 65) further comprises a coloured surface (120) which is exposed after actuation of the flexible structure (7, 12, 63, 64, 90).

14. Sensor system (1, 60) according to any of the preceding claims, wherein the second fluid system (8, 65) contains a dry colour inducing substance, which is dissolved by the target substance (T) or by a chemical added to the target substance after actuation of the flexible structure (7, 12, 63, 64, 90).

15. Sensor system (1, 60) according to any of the preceding claims, wherein the second fluid system (8, 65) contains an indicator substance (11, 32, 93), which reacts with the target substance (T) or by a chemical added to the target substance after actuation of the flexible structure (7, 12, 63, 64, 90) resulting in a sedimentation (110).

16. Sensor system (1, 60) according to any of the preceding claims, wherein the second fluid system (8, 65) contains an indicator substance (11, 32, 93), which reacts with the target substance (T) or with a chemical added to the target substance after actuation of the flexible structure (7, 12, 63, 64, 90) resulting in a colouring of a substance.

17. Sensor system (1, 60) According to any of the preceding claims, wherein the first fluid system (2, 61, 62) further comprises a filter (100) upstream of the flexible structure (7, 12, 63, 64, 90), the filter affecting the flow of the target substance (T) and/or any released fluid from the second fluid system (8, 65).

18. Sensor system (1, 60) according to any of the preceding claims, wherein the system comprises an array (150) of flexible structures (7, 12, 63, 64, 90).

19. Sensor system (1, 60) according to claim 18, wherein at least two of the flexible structures (7, 12, 63, 64, 90) in the array (150) are provided with different mechanical properties.

20. Sensor system (1, 60) according to claim 18, wherein at least two of the flexible structures (7, 12, 63, 64, 90) in the array (150) are provided with different chemical properties.

21. Sensor system (1, 60) according to any of the preceding claims, wherein the indicator substance (11, 32, 93) is an ink.

22. Sensor system (1, 60) according to any of the preceding claims, wherein the target substance (T) is an acid.

23. Sensor system (1, 60) according to any of the preceding claims, wherein the indicator substance (11, 32, 93) is contained in a reservoir (55) separate from the second fluid system (8, 65).

24. Method of sensing the presence of at least one target substance (T) in a fluid, the target substance being provided in an at least first fluid system (2, 61, 62), the first fluid system being separated from a second fluid system (8, 65) by a flexible structure (7, 12, 63, 64, 90),
wherein the second fluid system contains an indicator substance (11, 32, 93), the indicator substance being a coloured substance or a colour inducing substance, the flexible structure having a first surface (9, 66) facing the at least first fluid system, the first surface being functionalized to interact with the at least one target substance in a fluid present in the at least first fluid system, the flexible structure having a second surface (10, 67) facing the second fluid system, and
wherein the flexible structure upon exposure to the target substance releases energy stored in the flexible structure resulting in a mechanical actuation (MA) of the flexible structure, the mechanical actuation of the flexible structure resulting in a release of the coloured indicator substance either into the first fluid system or into a third fluid (68, 71, 80, 94) system connected to the second fluid system, or resulting in a colouring of a substance released either into the first fluid system or into a third fluid system connected to the second fluid system.

25. A process for manufacturing the sensor system (1, 60) according to claim 1, the process comprising the steps of
- providing a layer of a first material on a first substrate,
- masking the first material to indicate a pattern,
- defining the pattern by way of etching or developing to form at least one flexible structure (7, 12, 63, 64, 90) and at least a first fluid system (2, 61, 62), the flexible structure having a first (9, 66) and a second surface (10, 67), thereby forming a fluidic system and
- providing a second substrate of a second material,
- providing at least a second fluid system (8, 65) in the second substrate,
- providing an indicator substance (11, 32, 93) into the second fluidic system, the indicator substance being a coloured substance or a colour inducing substance, and
combining the fluidic system and second substrate.

26. A process according to claim 25 further comprising the step of providing a third fluid system (68, 71, 80, 94) in the second substrate.

27. A process according to any of the claims 25-26, wherein the first material is a radiation definable polymer, and wherein the process further comprises the steps of
- patterning the radiation definable polymer through exposure to radiation,
- developing the patterned radiation definable polymer to form at least one flexible structure (7, 12, 63, 64, 90) and at least a first fluid system (2, 61, 62), thereby forming a fluidic system.

28. A process according to claim 25, the process being a moulding technique wherein the first material is provided into a mould so that at least part of the at least first fluid system (2, 61, 62) is formed in one piece.

29. A process according to claim 25, the process being a moulding technique wherein the second material is provided into a mould so that at least part of the at least second fluid system (8, 65) and/or the at least third fluid system (68, 71, 80, 94) are formed in one piece.

30. A process according to any of the claims 25-29 further comprising the step of providing an indicator substance (11, 32, 93) into the second fluid system (8, 65)

31. Use of the sensor system (1, 60) according to claim 1, wherein the sensor system is used for detecting the presence of a chemical substance, a biological substance or a pharmaceutical substance in a gaseous ambient.

32. Use of the sensor system (1, 60) according to claim 1, wherein the sensor system is used for detecting the presence of a chemical substance, a biological substance or a pharmaceutical substance in a liquid environment.

33. Use of the sensor system (1, 60) according to claim 1, wherein the sensor system is a disposable item.

## Patentansprüche

1. Sensorsystem (1, 60), umfassend:
mindestens ein erstes Fluidsystem (2, 61, 62),
ein zweites Fluidsystem (8, 65) mit einer Indikatorsubstanz (11, 32, 93), wobei die Indikatorsubstanz eine gefärbte Substanz oder eine Farbe induzierende Substanz ist,
eine flexible Struktur (7, 12, 63, 64, 90), die das mindestens erste Fluidsystem und das zweite Fluidsystem trennt, wobei die flexible Struktur eine erste Fläche (9, 66) aufweist, die dem mindestens ersten Fluidsystem zugekehrt ist, wobei die erste Fläche (9, 66) derart funktionalisiert ist, dass sie mit mindestens einer Zielsubstanz (T) in einem in dem mindestens ersten Fluidsystem vorhandenen Fluid in Wechselwirkung tritt, wobei die flexible Struktur eine zweite Fläche (10, 67) aufweist, die dem zweiten Fluidsystem zugekehrt ist,
wobei die flexible Struktur nach Exposition an die Zielsubstanz Energie (ER) freisetzt, die in der flexiblen Struktur gespeichert war, was zu einer mechanischen Betätigung (MA) der flexiblen Struktur führt, wobei die mechanische Betätigung der flexiblen Struktur zu einer Freisetzung (RI) der gefärbten Indikatorsubstanz entweder in das erste Fluidsystem oder in ein drittes mit dem zweiten Fluidsystem verbundenes Fluidsystem (68, 71, 80, 94) führt oder zu einer Färbung einer Substanz führt, die in das erste Fluidsystem oder in ein drittes mit dem zweiten Fluidsystem verbundenes Fluidsystem freigesetzt wurde.

2. Sensorsystem (1, 60) nach Anspruch 1, wobei die Betätigung der flexiblen Struktur (7, 12, 63, 64, 90) weiterhin von einem physikalischen Auslöser bedingt ist.

3. Sensorsystem (1, 60) nach einem der vorhergehenden Ansprüche, wobei der physikalische Auslöser eine vorbestimmte Temperatur der Zielsubstanz (T), ein vorbestimmter Druck der Zielsubstanz und/oder ein Strahlungseinfall auf die Zielsubstanz und/oder die flexible Struktur (7, 12, 63, 64, 90) ist.

4. Sensorsystem (1, 60) nach einem der vorhergehenden Ansprüche, wobei die in der flexiblen Struktur (7, 12, 63, 64, 90) gespeicherte Energie durch Induzieren einer Veränderung der Oberflächenspannung der flexiblen Struktur freigesetzt wird.

5. Sensorsystem (1, 60) nach Anspruch 4, wobei die Veränderung der Oberflächenspannung der flexiblen Struktur (7, 12, 63, 64, 90) durch Ätzen einer oder mehrerer Überzugsschichten (30) erhalten wird.

6. Sensorsystem (1, 60) nach einem der vorhergehenden Ansprüche, wobei die erste Fläche (9, 66) mit einer oder mehreren Metallschichten, einer oder mehreren Molekularschichten und/oder einer oder mehreren Salzschichten überzogen ist.

7. Sensorsystem (1, 60) nach Anspruch 6, wobei die flexible Struktur (7, 12, 63, 64, 90) vor dem Beschichten der ersten Fläche (9, 66) vorbelastet wird.

8. Sensorsystem (1, 60) nach einem der vorhergehenden Ansprüche, wobei die zweite Fläche (10, 67) mit einer oder mehreren Metallschichten, einer oder mehreren Molekularschichten und/oder einer oder mehreren Salzschichten überzogen ist.

9. Sensorsystem (1, 60) nach Anspruch 6, wobei die eine oder mehreren Metallschichten mit einer Schutzschicht überzogen sind.

10. Sensorsystem (1, 60) nach einem der vorhergehenden Ansprüche, wobei die erste Fläche (9, 66) und/oder die zweite Fläche (10, 67) mit Teilchen im Nanogrößenbereich oder mit Teilchen im Mikrogrößenbereich überzogen ist/sind.

11. Sensorsystem (1, 60) nach einem der vorhergehenden Ansprüche, wobei die Auslenkung der flexiblen Struktur (7, 12, 63, 64, 90) im Bereich von 100 nm bis 1 mm liegt.

12. Sensorsystem (1, 60) nach einem der vorhergehenden Ansprüche, wobei die flexible Struktur (7, 12, 63, 64, 90) eine Polymerbasis aufweist.

13. Sensorsystem (1, 60) nach einem der vorhergehenden Ansprüche, wobei das zweite Fluidsystem (8, 65) weiterhin eine gefärbte Fläche (120) aufweist, die nach der Betätigung der flexiblen Struktur (7, 12, 63, 64, 90) exponiert ist.

14. Sensorsystem (1, 60) nach einem der vorhergehenden Ansprüche, wobei das zweite Fluidsystem (8, 65) eine Farbe induzierende Trockensubstanz enthält, die durch die Zielsubstanz (T) oder durch eine Chemikalie, die der Zielsubstanz nach Betätigung der flexiblen Struktur (7, 12, 63, 64, 90) zugesetzt wird, gelöst wird.

15. Sensorsystem (1, 60) nach einem der vorhergehenden Ansprüche, wobei das zweite Fluidsystem (8, 65) eine Indikatorsubstanz (11, 32, 93) enthält, die mit der Zielsubstanz (T) oder mit einer Chemikalie, die der Zielsubstanz nach Betätigung der flexiblen Struktur (7, 12, 63, 64, 90) zugesetzt wird, unter Bildung eines Niederschlags (110) reagiert.

16. Sensorsystem (1, 60) nach einem der vorhergehenden Ansprüche, wobei das zweite Fluidsystem (8, 65) eine Indikatorsubstanz (11, 32, 93) enthält, die mit der Zielsubstanz (T) oder mit einer Chemikalie, die der Zielsubstanz nach Betätigung der flexiblen Struktur (7, 12, 63, 64, 90) zugesetzt wird, unter Färbung einer Substanz reagiert.

17. Sensorsystem (1, 60) nach einem der vorhergehenden Ansprüche, wobei das erste Fluidsystem (2, 61, 62) weiterhin ein der flexiblen Struktur (7, 12, 63, 64, 90) vorgeschaltetes Filter (100) aufweist, wobei das Filter den Durchfluss der Zielsubstanz (T) und/oder jedes aus dem zweiten Fluidsystem (8, 65) freigesetzten Fluids beeinflusst.

18. Sensorsystem (1, 60) nach einem der vorhergehenden Ansprüche, wobei das System eine Gruppe (150) flexibler Struktur (7, 12, 63, 64, 90) aufweist.

19. Sensorsystem (1, 60) nach Anspruch 18, wobei mindestens zwei der flexiblen Strukturen (7, 12, 63, 64, 90) der Gruppe (150) mit unterschiedlichen mechanischen Eigenschaften ausgestattet sind.

20. Sensorsystem (1, 60) nach Anspruch 18, wobei mindestens zwei der flexiblen Strukturen (7, 12, 63, 64, 90) der Gruppe (150) mit unterschiedlichen chemischen Eigenschaften ausgestattet sind.

21. Sensorsystem (1, 60) nach einem der vorhergehenden Ansprüche, wobei die Indikatorsubstanz (11, 32, 93) eine Tinte ist.

22. Sensorsystem (1, 60) nach einem der vorhergehenden Ansprüche, wobei die Zielsubstanz (T) eine Säure ist.

23. Sensorsystem (1, 60) nach einem der vorhergehenden Ansprüche, wobei die Indikatorsubstanz (11, 32, 93) in einem Behälter (55) enthalten ist, der vom zweiten Fluidsystem (8, 65) getrennt ist.

24. Verfahren zum Erfassen der Gegenwart mindestens einer Zielsubstanz (T) in einem Fluid, wobei die Zielsubstanz in einem mindestens ersten Fluidsystem (2, 61, 62) bereitgestellt wird, wobei das erste Fluidsystem durch eine flexible Struktur (7, 12, 63, 64, 90) von einem zweiten Fluidsystem (8, 65) getrennt ist,
wobei das zweite Fluidsystem eine Indikatorsubstanz (11, 32, 93) enthält, wobei die Indikatorsubstanz eine gefärbte Substanz oder eine Farbe induzierende Substanz ist, wobei die flexible Struktur eine erste Fläche (9, 66) aufweist, die dem mindestens ersten Fluidsystem zugekehrt ist, wobei die erste Fläche (9, 66) derart funktionalisiert ist, dass sie mit der mindestens einen Zielsubstanz in einem in dem mindestens ersten Fluidsystem vorhandenen Fluid in Wechselwirkung tritt, wobei die flexible Struktur eine zweite Fläche (10, 67) aufweist, die dem zweiten Fluidsystem zugekehrt ist, und
wobei die flexible Struktur nach Exposition an die Zielsubstanz Energie freisetzt, die in der flexiblen Struktur gespeichert war, was zu einer mechanischen Betätigung (MA) der flexiblen Struktur führt, wobei die mechanische Betätigung der flexiblen Struktur zu einer Freisetzung der gefärbten Indikatorsubstanz entweder in das erste Fluidsystem oder in ein drittes mit dem zweiten Fluidsystem verbundenes Fluidsystem (68, 71, 80, 94) führt oder zu einer Färbung einer Substanz führt, die in das erste Fluidsystem oder in ein drittes mit dem zweiten Fluidsystem verbundenes Fluidsystem freigesetzt wurde.

25. Verfahren zur Herstellung des Sensorsystems (1, 60) nach Anspruch 1, wobei das Verfahren folgende Schritte umfasst
- Bereitstellen einer Schicht eines ersten Materials auf einem ersten Substrat,
- Maskieren des ersten Materials zur Angabe eines Musters,
- Festlegen des Musters durch Ätzen oder Entwickeln unter Bildung mindestens einer flexiblen Struktur (7, 12, 63, 64, 90) und mindestens eines ersten Fluidsystems (2, 61, 62), wobei die flexible Struktur eine erste (9, 66) und eine zweite Fläche (10, 67) aufweist, wodurch ein fluidisches System gebildet wird, und
- Bereitstellen eines zweiten Substrats eines zweiten Materials,
- Bereitstellen mindestens eines zweiten Fluidsystems (8, 65) in dem zweiten Substrat,
- Bereitstellen einer Indikatorsubstanz (11, 32, 93) in dem zweiten Fluidsystem, wobei die Indikatorsubstanz eine gefärbte Substanz oder eine Farbe induzierende Substanz ist, und
Vereinen des fluidischen Systems und des zweiten Substrats.

26. Verfahren nach Anspruch 25, weiterhin umfassend den Schritt des Bereitstellens eines dritten Fluidsystems (68, 71, 80, 94) in dem zweiten Substrat.

27. Verfahren nach einem der Ansprüche 25-26, wobei das erste Material ein durch Strahlung fixierbares Polymer ist und wobei das Verfahren weiterhin folgende Schritte umfasst
- Mustern des durch Strahlung fixierbaren Polymers durch Bestrahlung,
- Entwickeln des gemusterten durch Strahlung fixierbaren Polymers unter Bildung mindestens einer flexiblen Struktur (7, 12, 63, 64, 90) und mindestens eines ersten Fluidsystems (2, 61, 62), wodurch ein fluidisches System gebildet wird.

28. Verfahren nach Anspruch 25, wobei das Verfahren eine Formtechnik ist, wobei das erste Material in einer Form bereitgestellt wird, sodass mindestens ein Teil des mindestens ersten Fluidsystems (2, 61, 62) einstückig ausgebildet wird.

29. Verfahren nach Anspruch 25, wobei das Verfahren eine Formtechnik ist, wobei das zweite Material in einer Form bereitgestellt wird, sodass mindestens ein Teil des mindestens zweiten Fluidsystems (8, 65) und/oder des mindestens dritten Fluidsystems (68, 71, 80, 94) einstückig ausgebildet werden.

30. Verfahren nach einem der Ansprüche 25-29, ferner umfassend den Schritt des Bereitstellens einer Indikatorsubstanz (11, 32, 93) im zweiten Fluidsystem (8, 65).

31. Verwendung des Sensorsystems (1, 60) nach Anspruch 1, wobei das Sensorsystem zum Erfassen der Gegenwart einer chemischen Substanz, einer biologischen Substanz oder einer pharmazeutischen Substanz in einer gasförmigen Umgebung verwendet wird.

32. Verwendung des Sensorsystems (1, 60) nach Anspruch 1, wobei das Sensorsystem zum Erfassen der Gegenwart einer chemischen Substanz, einer biologischen Substanz oder einer pharmazeutischen Substanz in einer flüssigen Umgebung verwendet wird.

33. Verwendung des Sensorsystems (1, 60) nach Anspruch 1, wobei das Sensorsystem ein Einweg-Artikel ist.

## Revendications

1. Système de capteur (1, 60) comprenant :
au moins un premier système de fluide (2, 61, 62),
un deuxième système de fluide (8, 65) contenant une substance indicatrice (11, 32, 93), la substance indicatrice étant une substance colorée ou une substance induisant une coloration,
une structure souple (7, 12, 63, 64, 90) séparant l'au moins premier système de fluide du deuxième système de fluide, la structure souple possédant une première surface (9, 66) située face à l'au moins premier système de fluide, la première surface (9, 66) étant fonctionnalisée de manière à interagir avec au moins une substance cible (C) dans un fluide présent dans l'au moins premier système de fluide, la structure souple possédant une deuxième surface (10, 67) située face au deuxième système de fluide,
dans lequel la structure souple, lorsqu'elle est exposée à la substance cible, libère l'énergie (LE) stockée dans la structure souple, produisant un actionnement mécanique (AM) de la structure souple, l'actionnement mécanique de la structure souple produisant la libération (LI) de la substance indicatrice colorée soit dans le premier système de fluide, soit dans un troisième système de fluide (68, 71, 80, 94) raccordé au deuxième système de fluide, ou produisant une coloration d'une substance libérée soit dans le premier système de fluide, soit dans un troisième système de fluide raccordé au deuxième système de fluide.

2. Système de capteur (1, 60) selon la revendication 1, dans lequel l'actionnement de la structure souple (7, 12, 63, 64, 90) est également conditionné par un déclencheur physique.

3. Système de capteur (1, 60) selon l'une quelconque des revendications précédentes,
dans lequel le déclencheur physique est une température prédéterminée de la substance cible (C), est une pression prédéterminée de la substance cible et/ou est un rayonnement incident sur la substance cible et/ou la structure souple (7, 12, 63, 64, 90).

4. Système de capteur (1, 60) selon l'une quelconque des revendications précédentes,
dans lequel l'énergie stockée dans la structure souple (7, 12, 63, 64, 90) est libérée par le déclenchement d'une modification de la tension de surface de la structure souple.

5. Système de capteur (1, 60) selon la revendication 4, dans lequel la modification de la tension de surface de la structure souple (7, 12, 63, 64, 90) est obtenue par attaque chimique d'une ou de plusieurs couches de revêtement (30).

6. Système de capteur (1, 60) selon l'une quelconque des revendications précédentes,
dans lequel la première surface (9, 66) est revêtue d'une ou de plusieurs couches de métal, d'une ou de plusieurs couches moléculaires et/ou d'une ou de plusieurs couches de sel.

7. Système de capteur (1, 60) selon la revendication 6, dans lequel avant l'application du revêtement sur la première surface (9, 66), la structure souple (7, 12, 63, 64, 90) est préchargée.

8. Système de capteur (1, 60) selon l'une quelconque des revendications précédentes,
dans lequel la deuxième surface (10, 67) est revêtue d'une ou de plusieurs couches de métal, d'une ou de plusieurs couches moléculaires et/ou d'une ou de plusieurs couches de sel.

9. Système de capteur (1, 60) selon la revendication 6, dans lequel la(les) couche(s) de métal est(sont) revêtue(s) d'une couche de protection.

10. Système de capteur (1, 60) selon l'une quelconque des revendications précédentes,
dans lequel la première surface (9, 66) et/ou la deuxième surface (10, 67) est(sont) revêtue(s) de nanoparticules ou de microparticules.

11. Système de capteur (1, 60) selon l'une quelconque des revendications précédentes,
dans lequel la flexion de la structure souple (7, 12, 63, 64, 90) se situe dans la plage de 100 nm à 1 mm.

12. Système de capteur (1, 60) selon l'une quelconque des revendications précédentes,
dans lequel la structure souple (7, 12, 63, 64, 90) est polymérique.

13. Système de capteur (1, 60) selon l'une quelconque des revendications précédentes,
dans lequel le deuxième système de fluide (8, 65) comprend également une surface colorée (120) qui est exposée après l'actionnement de la structure souple (7, 12, 63, 64, 90).

14. Système de capteur (1, 60) selon l'une quelconque des revendications précédentes,
dans lequel le deuxième système de fluide (8, 65) comprend une substance sèche induisant une coloration, qui est dissoute par la substance cible (C) ou par une substance chimique ajoutée à la substance cible après l'actionnement de la structure souple (7, 12, 63, 64, 90).

15. Système de capteur (1, 60) selon l'une quelconque des revendications précédentes,
dans lequel le deuxième système de fluide (8, 65) contient une substance indicatrice (11, 32, 93), qui réagit avec la substance cible (C) ou avec une substance chimique ajoutée à la substance cible après l'actionnement de la structure souple (7, 12, 63, 64, 90), ce qui produit une sédimentation (110).

16. Système de capteur (1, 60) selon l'une quelconque des revendications précédentes,
dans lequel le deuxième système de fluide (8, 65) contient une substance indicatrice (11, 32, 93), qui réagit avec la substance cible (C) ou avec une substance chimique ajoutée à la substance cible après l'actionnement de la structure souple (7, 12, 63, 64, 90), ce qui produit une coloration d'une substance.

17. Système de capteur (1, 60) selon l'une quelconque des revendications précédentes,
dans lequel le premier système de fluide (2, 61, 62) comprend également un filtre (100) en amont de la structure souple (7, 12, 63, 64, 90), le filtre influant sur le débit de la substance cible (C) et/ou de tout fluide libéré par le deuxième système de fluide (8, 65).

18. Système de capteur (1, 60) selon l'une quelconque des revendications précédentes,
dans lequel le système comprend un réseau (150) de structures souples (7, 12, 63, 64, 90).

19. Système de capteur (1, 60) selon la revendication 18, dans lequel au moins deux des structures souples (7, 12, 63, 64, 90) du réseau (150) se voient conférer des propriétés mécaniques différentes.

20. Système de capteur (1, 60) selon la revendication 18, dans lequel au moins deux des structures souples (7, 12, 63, 64, 90) du réseau (150) se voient conférer des propriétés chimiques différentes.

21. Système de capteur (1, 60) selon l'une quelconque des revendications précédentes,
dans lequel la substance indicatrice (11, 32, 93) est une encre.

22. Système de capteur (1, 60) selon l'une quelconque des revendications précédentes,
dans lequel la substance cible (C) est un acide.

23. Système de capteur (1, 60) selon l'une quelconque des revendications précédentes,
dans lequel la substance indicatrice (11, 32, 93) est contenue dans un réservoir (55) séparé du deuxième système de fluide (8, 65).

24. Méthode de détection de la présence d'au moins une substance cible (C) dans un fluide, la substance cible étant apportée par au moins un premier système de fluide (2, 61, 62), le premier système de fluide étant séparé d'un deuxième système de fluide (8, 65) par une structure souple (7, 12, 63, 64, 90),
dans laquelle le deuxième système de fluide contenant une substance indicatrice (11, 32, 93), la substance indicatrice étant une substance colorée ou une substance induisant une coloration, la structure souple possédant une première surface (9, 66) située face à l'au moins premier système de fluide, la première surface étant fonctionnalisée de manière à interagir avec l'au moins une substance cible dans un fluide présent dans l'au moins premier système de fluide, la structure souple possédant une deuxième surface (10, 67) située face au deuxième système de fluide, et
dans laquelle la structure souple, lorsqu'elle est exposée à la substance cible, libère l'énergie stockée dans la structure souple, produisant un actionnement mécanique (AM) de la structure souple, l'actionnement mécanique de la structure souple produisant la libération de la substance indicatrice colorée soit dans le premier système de fluide, soit dans un troisième système de fluide (68, 71, 80, 94) raccordé au deuxième système de fluide, ou produisant une coloration d'une substance libérée soit dans le premier système de fluide, soit dans un troisième système de fluide raccordé au deuxième système de fluide.

25. Procédé de fabrication du système de capteur (1, 60) selon la revendication 1, le procédé comprenant les étapes suivantes :
- apport d'une couche d'un premier matériau sur un premier substrat,
- masquage du premier matériau pour indiquer un motif,
- définition du motif par attaque chimique ou par développement pour former au moins une structure souple (7, 12, 63, 64, 90) et au moins un premier système de fluide (2, 61, 62), la structure souple possédant une première (9, 66) et une deuxième surface (10, 67), formant ainsi un système fluidique et
- apport d'un deuxième substrat constitué d'un deuxième matériau,
- apport d'au moins un deuxième système de fluide (8, 65) dans le deuxième substrat,
- apport d'une substance indicatrice (11, 32, 93) dans le deuxième système fluidique, la substance indicatrice étant une substance colorée ou une substance induisant une coloration, et
combinaison du système fluidique et du deuxième substrat.

26. Procédé selon la revendication 25 comprenant également une étape d'apport d'un troisième système de fluide (68, 71, 80, 94) dans le deuxième substrat.

27. Procédé selon l'une quelconque des revendications 25 et 26, dans lequel le premier matériau est un polymère définissable par rayonnement, et dans lequel le procédé comprend également les étapes suivantes :
- production de motifs sur le polymère définissable par rayonnement par exposition à un rayonnement,
- développement du polymère définissable par rayonnement comportant des motifs pour former au moins une structure souple (7, 12, 63, 64, 90) et au moins un premier système de fluide (2, 61, 62), en formant ainsi un système fluidique.

28. Procédé selon la revendication 25, le procédé étant une technique de moulage dans laquelle le premier matériau est apporté dans un moule de manière à ce qu'au moins une partie de l'au moins premier système de fluide (2, 61, 62) soit formée d'un seul tenant.

29. Procédé selon la revendication 25, le procédé étant une technique de moulage dans laquelle le deuxième matériau est apporté dans un moule de manière à ce qu'au moins une partie de l'au moins deuxième système de fluide (8, 65) et/ou de l'au moins troisième système de fluide (68, 71, 80, 94) soit formée d'un seul tenant.

30. Procédé selon l'une quelconque des revendications 25 à 29 comprenant également une étape d'apport d'une substance indicatrice (11, 32, 93) dans le deuxième système de fluide (8, 65).

31. Utilisation du système de capteur (1, 60) selon la revendication 1, dans laquelle le système de capteur est utilisé pour détecter la présence d'une substance chimique, d'une substance biologique ou d'une substance pharmaceutique dans un environnement gazeux.

32. Utilisation du système de capteur (1, 60) selon la revendication 1, dans laquelle le système de capteur est utilisé pour détecter la présence d'une substance chimique, d'une substance biologique ou d'une substance pharmaceutique dans un environnement liquide.

33. Utilisation du système de capteur (1, 60) selon la revendication 1, dans laquelle le système de capteur est un élément jetable.
